# EUROPEAN PATENT APPLICATION

(11) **EP 0 990 441 A1**
(43) Date of publication of application: **05.04.2000**
(21) Application number: 98941214.3
(22) Date of filing: 01.09.1998
(51) Int. Cl.: A61K 31/185

(54) **A DRUG FOR TREATING DIABETIC NEPHROSIS**

(30) Priority: 30.09.1997 CN 97107137
(71) Applicant: Nanjing General Hospital of Nanjing Command PLA, Nanjing, Jiang Su 210002 (CN)
(72) Inventor: LI, Leishi, Nanjing City Jiang Su Province 210002 (CN)
(74) Representative: Stalla-Bourdillon, Bernard
(86) International application number: CN9800175
(87) International publication number: WO9916432

(57) **Abstract**

A medicine for treatment of diabetic nephropathy contains rhein or the salt thereof. It may be supplied in aqueous solution or capsula for oral, such dosage is not larger than 200mg/day. Toxicity of rhein or the salt thereof is low, LD50 is 3.2g/kg. The medicine concentration in blood is still higher in 24 hours after administered rhein by oral. Rhein could effectively control or decrease the hyperglycemia, suppress renal hypertrophy and kidney index in experimental diabetic rats. The urinary albumin execretion was significantly decreased in the diabetic patients treated by rhein.

## Description

### TECHNICAL FIELD

The present invention relates to an anthraquinone carboxylic acid, in particular, to a medicine containing rhein for treatment of diabetic nephropathy.

### BACKGROUND OF THE INVENTION

Diabetes is a frequently occurring disease, and the main medicament for its treatment is insulin at present. However, insulin has no therapeutic effect to the kidney damage caused by diabetes, such as renal hypertrophy, although it can decrease the urinary glucose. Until now, no report has been found on the medicine for treatment of renal hypertrophy caused by diabetes.

### DESCRIPTION OF THE INVENTION

The object of the present invention is to provide a medicine of low toxicity for treatment of diabetic nephropathy.

The technical solution of the present invention is to prepare an oral medicine for treatment of diabetic nephropathy from rhein or the salt thereof.

Rhein is a particular medicine in China, that is one of the most common used Chinese medicines. The general formula of rhein can be shown as the following:

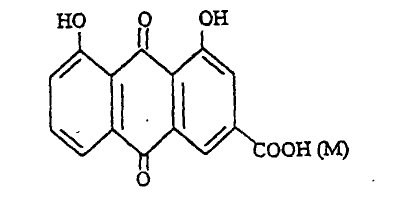

Rhein is one of the chemical components in rhubarb (*Rheum Officinale*, a Chinese herb) and of low toxicity as a natural product. The half lethal dose (LD₅₀) is 3.2g/Kg for SD rats.

There are not marked gastro-intestinal reactions caused by the oral route of rhein in volunteers treated by rhein. Of the thirty-six volunteers given rhein at dose of 200mg/day (100mg, b.i.d) for 3 days, only 5 persons experience gastro-intestinal disorders. Diarrhea occurred twice or thrice a day in three of these persons, and four times in only one person.

In pharmacokinetical study, rhein can be easily absorbed from gastro-intestinal trace after the agent is given orally, and the blood concentration of rhein maintains for a longer period. The blood level of rhein reaches peak in one and half hours to three hours after one single dose of rhein at 200mg in health man, and the blood level of them still kept at 200 to 500 ng/ml at 24th hour in volunteers.

Thus, rhein possesses advantageous as medicine.

As shown in further investigation of rhein, hyperglycemia in experimental diabetic rats can be controlled or reduced effectively by rhein or sodium rheinate given orally. Particularly rhein is able to control or suppress renal hypertrophy, and decrease the kidney index obviously in diabetic rats. Rhein also can ameliorate the urinary albumin excretion significantly in diabetic nephropathy patients.

The medicine containing rhein or the salt thereof for treatment of diabetic nephropathy may be supplied in oral capsula or aqueous solution.

A dosage of rhein up to 200 mg/day is feasible, and may be given twice or thrice a day.

The following Examples will illustrate the present invention in more details.

### EXAMPLE 1

### Suppression of renal hypertrophy by rhein in experiment diabetic rats

Drug: Rhein, a needle-like yellow crystal with melting point of 318-320°C, was prepared into aqueous solution for oral.

Diabetic animal model of rat: Adult female SD rats with body weights of 200 to 250 grams were used. Rats were made diabetic by an intra-abdominal injection of Streptozotocin in a dose of 25 mg/Kg/day for 5 days. Only rats with blood glucose level within the range of 13 to 25 mmol/L were recognized as the establishment of the diabetic model.

The observation was focused on the changes of kidney weight and volume in diabetic rats treated by rhein.

The experiment was designed as the following:

| | Normal rats | | Diabetic rats | |
|---|---|---|---|---|
| | Control | Rhein-treated | Control | Rhein-treated |
| No. of animals | 8 | | 7 | 6 |
| Dosage of Rhein: mg/Kg/day | 0 | 2 | 0 | 2 |
| Distilled water: ml/Kg/day | 5 | 5 | 5 | 5 |

The experimental results are listed in the following tables.

### 1. Effect of rhein on the changes of the blood glucose levels (mmol/L)

| | Normal rats | | Diabetic rats | |
|---|---|---|---|---|
| | Control | Rhein-treated | Control | Rhein-treated |
| Pre-diabetic | 6.62±0.84 | 6.12±0.34 | 5.62±1.84 | 6.23±0.44 |

| After diabetic(weeks) | | | | |
|---|---|---|---|---|
| 1 | 6.00±0.44 | 5.92±0.54 | 19.82±2.84 | 20.32±1.89 |
| 4 | 6.31±0.36 | 5.66±0.84 | 21.12±2.19 | 19.77±3.87 |
| 8 | 5.82±0.39 | 6.07±0.49 | 19.92±3.23 | 21.00±3.26 |
| 12 | 6.02±0.51 | 6.22±0.74 | 20.62±0.64 | 19.32±1.72 |

### 2. Effect of rhein on the kidney weight at the 12th week

| | Normal rats | | Diabetic rats | |
|---|---|---|---|---|
| | Control | Rhein-treated | Control | Rhein-treated |
| Kidney Weight (grams) | 0.84±0.09 | 0.72±0.11 | 1.31±0.73 | 0.93±0.13 |
| Kidney Volume (ml) | 0.794±0.019 | 0.804±0.210 | 1.083±0.049 | 0.834±0.109 |
| Kidney Index (× 10⁻³) | 3.33±0.21 | 4.04±0.39 | 7.21±1.30 | 4.76±0.76 |

### EXAMPLE 2

### Suppression of renal hypertrophy by sodium rheinate in experiment diabetic rats

Drag: aqueous solution of sodium rheinate for oral

Diabetic animal model of rat: Adult female SD rats with body weights of 200 to 250 grams were used. Rats were made diabetic by an intra-abdominal injection of Streptozotocin in a dose of 25 mg/Kg/day for 5 days. Only rats with blood glucose level in the range of 13 to 25 mmol/L were recognized as the establishment of the diabetic mode.

The observation was focused on the changes of kidney weight and volume in diabetic rats treated by rhein.

The experiment was designed as the following:

| | Normal rats | | Diabetic rats | |
|---|---|---|---|---|
| | Control | Rhein-treated | Control | Rhein-treated |
| No. of animals | 6 | 5 | 6 | 6 |
| Dosage of Rhein-Na, mg/Kg/day | 0 | 1 | 0 | 1 |
| Distilled water, ml/Kg/day | 5 | 5 | 5 | 5 |

The experimental results are listed in the following tables.

### 1. Effect of sodium rheinate on the changes of the blood glucose levels (mmol/L)

| | Normal rats | | Diabetic rats | |
|---|---|---|---|---|
| | Control | Rhein-treated | Control | Rhein-treated |
| Pre-diabetic | 5.33±0.54 | 6.06±0.72 | 6.12±0.84 | 6.17±1.01 |

| After diabetic (weeks) | | | | |
|---|---|---|---|---|
| 1 | 6.22±0.74 | 6.52±0.74 | 22.16±3.64 | 21.82±2.69 |
| 4 | 5.88±0.66 | 6.48±0.77 | 21.57±3.99 | 19.97±4.17 |

### 2. Effect of sodium Rheinate on the kidney weight (g) at the 12th week

| | Normal rats | | Diabetic rats | |
|---|---|---|---|---|
| | Control | Rhein-treated | Control | Rhein-treated |
| Kidney Weight (grams) | 0.66±0.11 | 0.59±0.09 | 0.89±0.22 | 0.71±0.20 |
| Kidney Index (× 10⁻³) | 4.13±0.09 | 4.34±0.37 | 7.94±1.73 | 5.68±1.16 |

### EXAMPLE 3

### Clinical trial of rhein on diabetic nephropathy

Patients: Twenty-five diabetic patients diagnosed according to the standard of WHO were enrolled in this study. The fast blood glucose levels were above 250mg/100ml in these patients. Diabetic nephropathy was confirmed morphologically by renal biopsy, or with measurement of urinary protein or albumin excretion in each patient.

Design: A yellow powder supplied in capsula containing 25mg rhein. The dosage of 100 mg per day (50mg twice a day) rhein was given orally as therapy. All patients were followed at regular intervals for measurement of serum creatinine values and urinary protein or albumin excretion at least for 3 months.

The experimental results are listed as the following:

### 1. General clinical data

All patients enrolled into this study tolerated rhein treatment. None of the recipients receiving rhein at dose of 100 mg per day had gastro-intestinal reactions as vomiting, diarrhea et al.

There are no liver function abnormally and deterioration of renal function in patients treated with rhein. Blood routine test is normal.

### 2. Changes of urinary protein excretion

Of twenty-five diabetic patients given rhein treatment, 18 patients (72%) had obvious decrement of urinary albumin excretion during the 3 months therapy. There are seven diabetic patients (28%) with no change or increase of urinary albumin excretion. The datum are shown in the following table:

| Change of urinary albumin Excretion | Treatment after 3 months (No.) |
|---|---|
| No change | 7 |
| Decrease of 25% | 4 |
| Decrease of 50% | 9 |
| Decrease of 75% | 4 |

By clinical trial, rhein can decrease the urinary protein excretion in diabetic patients treated by rhein. It is suggested that the medicine possesses therapeutic effect on the kidney damage in diabetic nephropathy, and can be used in clinical practice of diabetes.

### INDUSTRIAL APPLICATION

The medicine provide by the present invention can be absorbed easily from gastrointestinal trace, decrease the urinary protein excretion in diabetic patients treated, obviously reduce kidney index. It can be prepared into oral capsula or oral aqueous solution for the clinical treatment of diabetic nephropathy.

## Claims

1. A medicine for treatment of diabetic nephropathy, wherein comprising rhein or the salt thereof.

2. A medicine for treatment of diabetic nephropathy in accordance with claim 1, wherein the said rhein salt is sodium rheinate or potassium rheinate.

3. A medicine for treatment of diabetic nephropathy in accordance with claim 1, wherein it is supplied in capsule or solution for oral.

4. A medicine for the treatment of diabetic nephropathy in accordance with claim 1, wherein the daily dosage of rhein or the salt thereof is not larger than 200mg/day when given orally.
